(19) **Europäisches Patentamt European Patent Office Office européen des brevets**

(11) **EP 1 764 036 B1**

(12) **EUROPEAN PATENT SPECIFICATION**

(45) Date of publication and mention
of the grant of the patent:
**10.09.2008 Bulletin 2008/37**

(51) Int Cl.:
***A61B 5/087*** (2006.01)

(21) Application number: **06019343.0**

(22) Date of filing: **15.09.2006**

(54) **Method for the determination of the time-delay between a main-stream ultrasonic flow sensor and a side-stream gas analyzer**

Verfahren zur Zeitverzögerungsmessung zwischen einen Ultraschallhauptströmungsmessgerät und einen Seitenstromgasanalysator

Méthode de détermination du retard entre un capteur de débit ultrasonique placé dans un courant principal et un analyseur de gaz placé dans un courant secondaire

| | |
|---|---|
| (84) Designated Contracting States:<br>**AT BE BG CH CY CZ DE DK EE ES FI FR GB GR HU IE IS IT LI LT LU LV MC NL PL PT RO SE SI SK TR** | (72) Inventors:<br>• **Buess, Christian**<br>  **8810 Horgen (CH)**<br>• **Harnoncourt, Georg**<br>  **8008 Zürich (CH)** |
| (30) Priority: **16.09.2005 US 717700 P** | |
| | (74) Representative: **Laufhütte, Dieter et al**<br>**Lorenz-Seidler-Gossel**<br>**Widenmayerstrasse 23**<br>**80538 München (DE)** |
| (43) Date of publication of application:<br>**21.03.2007 Bulletin 2007/12** | |
| (73) Proprietor: **ndd Medizintechnik AG**<br>**8005 Zürich (CH)** | (56) References cited:<br>**EP-A2- 0 646 346       EP-A2- 1 112 716**<br>**EP-A2- 1 279 368       DE-A1- 10 352 652** |

## Description

In lung function diagnostics as well as in lung function monitoring the amount of gas flowing in and out the patient's lung has to be measured. This is usually accomplished by using gas flow meters (also called pneumotachographs) combined with specific gas analyzers. An example is exercise spirometry where the ventilation of patients is measured while performing exercise. In this case the oxygen uptake, i.e. the volume of oxygen consumed by the body, has to be determined. Oxygen uptake is computed by measuring flow velocity $F$ and oxygen concentration $f_{O2}$. By integrating flow velocity multiplied by the oxygen concentration over time, the in- and expiratory oxygen volume can be determined. Oxygen uptake is finally computed by subtracting the expiratory oxygen volume from the inspiratory oxygen volume.

Document EP 0 646 346 discloses a device consisting of an ultrasonic gas flow and molar mass sensors for medical application.

For most lung function measurements flow and gas concentrations are sampled at a frequency of approx. 100 to 200 Hz (sampling time 10 to 5 ms). In order to correctly perform the above mentioned integration of flow multiplied by the oxygen gas concentration the time-delay between the two signals should not exceed the sampling rate.

In current systems the delay between a main-stream flow sensor and a side-stream gas sensor is normally determined by one of the following ways:

> 1. Assuming a fixed time-delay calculated from the flow velocity of the side-stream and the dead space volume between main-stream and side-stream sensor. The delay calculated by this method assumes a constant velocity of the side-stream and a constant dead space. Due to several factors like water vapor condensation in the side-stream tubing and changing gas viscosities, both assumptions do not apply for most applications.

> 2. Introducing a well defined marker in the gas stream and measuring the time that the marker needs to be detected by the side-stream gas sensor.

The methods described either require additional hardware that needs to be introduced or they are not very reliable in daily use.

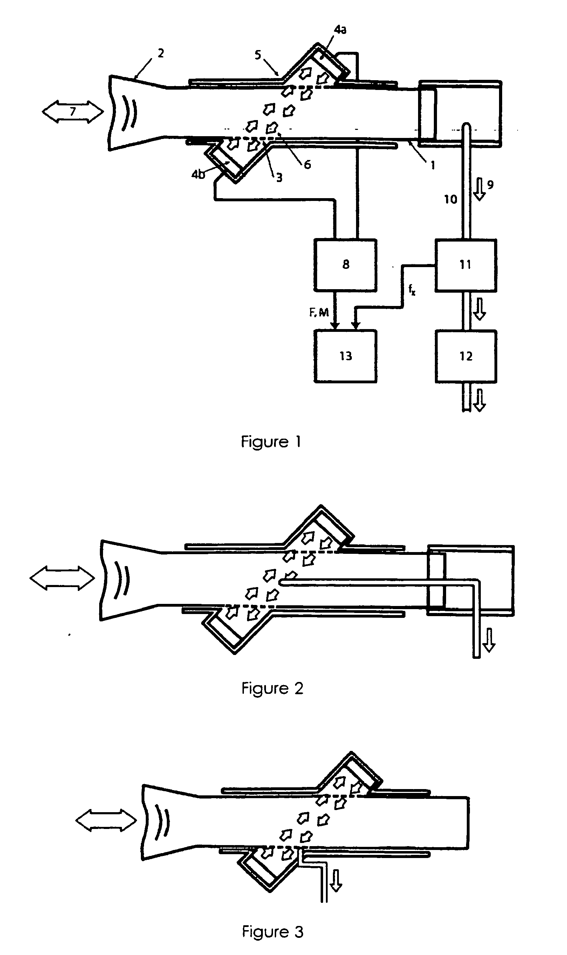

The invention presented can be used to automatically determine the time-delay between a main-stream flow sensor and one or several side-stream gas sensors in a reliable way. Figure 1 shows a block diagram of a system that can be used to determine oxygen uptake. The system consists of an ultrasonic flow sensor and a side-stream gas sensor. The operation of an ultrasonic flow meter is described in many publications (see [1], [2], [3]). The flow sensor consists of two ultrasonic transducers (4a, 4b) mounted on opposite sides of the gas flow (7), an appropriate case (5) and an exchangeable breathing tube (1) with attached mouth piece (2). The flow velocity is determined in a signal processing unit (8) using the transit-times (time-of-flight) of ultrasonic pulse trains (6) transmitted in up- and downstream direction of the gas flow. The pulse trains are transmitted and received by the ultrasonic transducers. The pulse trains travel along the sound transmission path through ultrasonically permeable parts (3, e.g. meshes, filters etc.) of the flow tube. Flow velocity is determined using the following equation:

$$F = k \frac{t_1 - t_2}{t_1 \cdot t_2},$$

where F is the velocity of the gas flow, $t_1$ and $t_2$ represent the transit-times in up-and downstream direction, and k is a constant that depends on the mechanical dimensions of the flow sensor.

At the end of the breathing tube (1) a small part of the main-stream flow is fed to a side-stream system. By using a gas pump (12) the side-stream flow (9) passes one or several gas analyzers (11). The tubing (9) between flow sensor and side-stream gas analyzer may consists of normal plastic tubing or of special tubing that equilibrates water vapor. Examples for side-stream sensors are sensors for oxygen or carbon dioxide that can be used for exercise spirometry data analysis, or sensors for helium or $CH_4$ used for the measurement of functional residual capacity (FRC) or diffusion capacity of the lung (DLCO). Figure 2 shows an alternative setup where the side-stream gas flow is sampled at exactly at the centre of the sound transmission path. This arrangement avoids differences in the time-delay caused by flow velocity and by flow direction.

Figure 3 shows another alternative setup where the side-stream flow is sampled through the side chamber of the ultrasonic flow sensor. The advantages of this method are the following: 1) Less contamination of the side-stream flow since the side-stream flow passes through the filter or mesh in front of the side chamber, and 2) no part that sticks into the main flow tube.

In addition to flow the processing unit of the ultrasonic flow sensor (Fig. 1, 8) can determine molar mass of the gas within the flow sensor. Molar mass is normally determined using the following equation:

$$M = k \cdot \kappa \cdot R \cdot T \cdot \left( \frac{t_1 \cdot t_2}{t_1 + t_2} \right)^2,$$

where M is the molar mass, T is the mean temperature along the sound transmission path, R is the gas constant, $\kappa$ is the relation of the specific heat capacities ($c_p/c_v$) of

the gas, k is a constant that depends on the mechanical dimensions of the sensor, and $t_1$ and $t_2$ represent the transit-times (see [2]). Temperature T can be determined by one or several temperature measurements along the sound transmission path; it can be determined by a combination of a temperature measurement and a mathematical model; or it can be a constant value.

In contrast to other methods using flow- and gas composition (respectively gas concentration) measurement the ultrasonic transit-time measurement determines the over all molar mass of the gas mixture and not the concentration of a specific gas. Flow and molar mass signals, however, are always time aligned, there is no time-delay between flow and molar mass signals.

In the present invention the time-delay between the side-stream gas concentration $f_x$ and flow F is determined by using information contained in the molar mass signal. For most gases used in respiratory gas analysis the waveform of the molar mass signal is similar to the waveform of the specific gas measured in the side-stream gas sensor. The time-delay between flow and gas signal from the side-stream sensor can now be determined by several methods:

- Mathematical cross correlation between molar mass signal and side-stream gas concentration measurement.

- Automatic detection of clearly defined points in the curve, e.g. the change of the gas concentration from inspiration to expiration, or the change from expiration to inspiration.

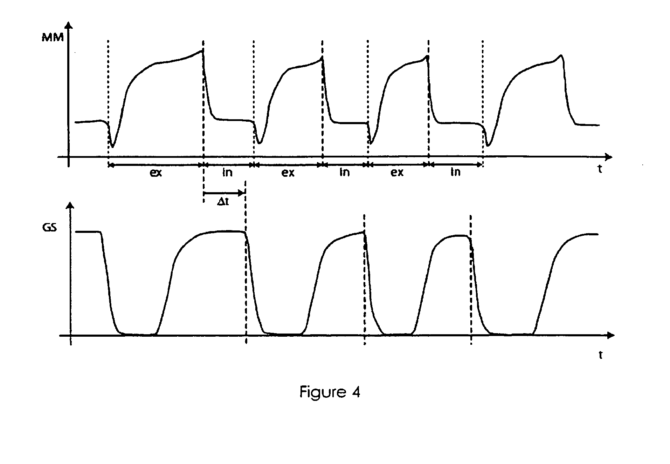

Using these methods the time-delay between molar mass and side-stream gas signal can be determined. Figure 4 shows the waveforms of main-stream molar mass signal (MM) and the side-stream gas signal (SG) over time and the time-delay ($\Delta t$) determined by the methods described above. Since flow main-stream and molar mass signals are synchronous, the time-delay corresponds to the time-delay between flow and side-stream gas concentration measurement.

The method described can be implemented in a standalone device, e.g. a hand held system, it can be implemented in a device used in intensive care units, e.g. in a ventilator that includes respiratory gas measurement, or it can alternatively be implemented in a computer based device, where an ultrasonic flow and molar mass sensor and one or several side-stream gas sensors are interfaced to a computer that is used for data analysis and data representation.

The time-delay can be computed on a breath by breath basis, or it can alternatively be determined a single time for a series of breaths or once per entire data acquisition.

**Claims**

1. Device consisting of an ultrasonic gas flow and molar mass sensor for medical application based on the transit-time or time-of-flight method, an exchangeable or fixed flow tube with mouth piece, combined with one or several side-stream gas sensors, i.e. gas sensors that are removed from main gas flow and are placed in a side-stream gas flow that transports only a fraction of the main-stream gas flow, **characterized in that** the time-delay between the output signal of the side-stream gas sensor and the main-stream flow signal is determined by correlating the molar mass signal to the signal from the side-stream gas sensor(s).

2. Device according to claim 1, where the time-delay is determined by mathematical cross correlation of the entire main-stream molar mass signal or by parts of the main-stream molar mass signal to corresponding parts of the side-stream gas sensor signal.

3. Device according to claim 2, where the time-delay is determined by correlating clearly defined points in the main-stream molar mass signal to the appropriate points in the side-stream gas signal(s), i.e. the start of inspiration or start of expiration.

4. Device according to claim 3, where the method uses native molar mass, where a fixed temperature is assumed for the molar mass computation, or normal molar mass by using a temperature model, one or several temperature measurements or a combination of both for the computation of the temperature along the sound transmission path.

5. Device according to claim 4, where the method is used for the analysis of exercise or stress testing.

6. Device according to claim 5, where the method is used for the analysis of functional residual capacity (FRC) or alveolar volume ($V_A$) or diffusion capacity of the lung (DLco).

7. Device according to claim 6, where the method is used for monitoring of patients, i.e. for intensive care applications or in anaesthesia.

8. Device according to claim 7, where external gas sensor is a side-stream ultrasonic flow and molar mass sensor or a combination of a gas sensor and an ultrasonic flow and molar mass sensor.

9. Device according to claim 8, where the gas sample is taken from the centre point of the flow and molar mass measurement path, therefore avoiding any additional time-delay differences caused by flow velocity and/or flow direction.

10. Device according to claim 8, where the gas sample is taken through the filter or net or mesh that covers the side chamber of the ultrasonic transducer mounting of the main-stream ultrasonic flow sensor, therefore avoiding any additional time-delay difference caused by flow velocity and/or flow direction and additionally reducing or avoiding contamination of the side-stream sensors.

**Patentansprüche**

1. Vorrichtung bestehend aus einem Ultraschall-Gasstrom- und Molmassensensor für medizinische Anwendung basierend auf der Laufzeitmethode, einem auswechselbaren oder befestigten Stromrohr mit Mundstück, kombiniert mit einem oder mehreren Seitenstrom-Gassensoren, z.B. Gassensoren, die vom Hauptgasstrom entfernt und in einem Seitenstrom-Gasstrom, der nur einen Bruchteil des Hauptstrom-Gasstroms transportiert, angeordnet sind, **dadurch gekennzeichnet, dass** die Zeitverzögerung zwischen dem Ausgabesignal des Seitenstrom-Gassensors und dem Hauptstrom-Stromsignal durch Korrelieren des Molmassensignals mit dem Signal von dem Seitenstrom-Gassensor / den Seitenstrom-Gassensoren ermittelt wird.

2. Vorrichtung nach Anspruch 1, wobei die Zeitverzögerung durch mathematische Kreuzkorrelation des gesamten Hauptstrom-Molmassensignals oder durch Teile des Hauptstrom-Molmassensignals zu entsprechenden Teilen des Seitenstrom-Gassensorsignals ermittelt wird.

3. Vorrichtung nach Anspruch 2, wobei die Zeitverzögerung durch Korrelieren klar definierter Punkte im Hauptstrom-Molmassensignal zu den geeigneten Punkten in dem Seitenstrom-Gassignal / den Seitenstrom-Gassignalen, z.B. dem Start der Einatmung oder dem Start der Ausatmung, ermittelt wird.

4. Vorrichtung nach Anspruch 3, wobei das Verfahren native Molmasse unter Annahme einer festgelegten Temperatur für die Molmassenberechnung oder normale Molmasse durch Verwenden eines Temperaturmodells, einer oder mehrerer Temperaturmessungen oder einer Kombination von beiden für die Berechnung der Temperatur entlang der Schallübertragungsstrecke nutzt.

5. Vorrichtung nach Anspruch 4, wobei das Verfahren für die Analyse von Übungs- oder Belastungstests verwendet wird.

6. Vorrichtung nach Anspruch 5, wobei das Verfahren für die Analyse der funktionellen Residualkapazität (FRS) oder des Alveolarvolumens ($V_A$), oder der Diffusionskapazität der Lunge ($DL_{CO}$) verwendet wird.

7. Vorrichtung nach Anspruch 6, wobei das Verfahren zum Überwachen von Patienten, d.h. für Intensivpflegeanwendungen oder in der Anästhesie, verwendet wird.

8. Vorrichtung nach Anspruch 7, wobei der externe Gassensor ein Ultraschall-Strom- und Molmassensensor des Seitenstroms oder eine Kombination aus einem Gassensor und einem Ultraschall-Strom- und Molmassensensor ist.

9. Vorrichtung nach Anspruch 8, wobei die Gasprobe aus dem Mittelpunkt der Strom- und Molmassenmeßstrecke entnommen wird, wodurch jegliche zusätzlichen durch Strömgeschwindigkeit und/oder Strömrichtung verursachten Zeitverzögerungsdifferenzen vermieden werden.

10. Vorrichtung nach Anspruch 8, wobei die Gasprobe durch den Filter oder das Netz oder das Sieb, die die Seitenkammer der Ultraschallwandlerhalterung des Hauptstrom-Ultraschallstromsensors abdecken, entnommen wird, wodurch jegliche zusätzliche durch Strömgeschwindigkeit und/oder Strömrichtung verursachte Zeitverzögerungsdifferenz vermieden wird und zusätzlich Verunreinigung der Seitenstrom-Sensoren gemindert oder vermieden wird.

**Revendications**

1. Dispositif constitué d'un capteur d'écoulement de gaz et de masse molaire ultrasonique pour une application médicale basé sur le procédé de temps de transit au temps de vol, d'un tube d'écoulement échangeable ou fixe avec un embout, combiné avec un ou plusieurs capteurs de gaz de courant latéral, c'est-à-dire de capteurs de gaz qui sont retirés de l'écoulement principal du gaz et sont placés dans un écoulement de gaz de courant latéral qui transforme seulement une fraction de l'écoulement de gaz du courant principal, **caractérisé en ce que** le temps de retard entre le signal de sortie du capteur de gaz d'écoulement latéral et le signal d'écoulement de courant principal est déterminé en mettant en corrélation un signal de masse molaire avec le signal du ou des capteurs de gaz de courant latéral.

2. Dispositif selon la revendication 1, où le temps de retard est déterminé par une corrélation mathématique croisée du signal de masse molaire de courant principal entier ou par des parties du signal de masse molaire de courant principal à des parties correspondantes du signal de capteur de gaz de courant latéral.

**3.** Dispositif selon la revendication 2, où le temps de retard est déterminé en mettant en corrélation des points définis clairement dans le signal de masse molaire de courant principal aux points appropriés dans le ou les signaux de gaz de courant latéral, c'est-à-dire le début d'inspiration ou le début d'expiration.

**4.** Dispositif selon la revendication 3, où le procédé utilise une masse molaire native, où une température fixe est supposée pour le calcul de la masse molaire, ou bien une masse molaire normale en utilisant un modèle de température, une ou plusieurs mesures de température ou une combinaison des deux pour le calcul de la température le long du chemin de transmission du son.

**5.** Dispositif selon la revendication 4, où le procédé est utilisé pour l'analyse de tests se rapportant à des exercices ou à l'effort.

**6.** Dispositif selon la revendication 5, où le procédé est utilisé pour l'analyse de la capacité résiduelle fonctionnelle (FRC) ou du volume alvéolaire ($V_A$) ou de la capacité de diffusion du poumon ($DL_{CO}$).

**7.** Dispositif selon la revendication 6, où le procédé est utilisé pour surveiller des patients, par exemple pour des applications de soins intensifs ou pour l'anesthésie.

**8.** Dispositif selon la revendication 7, où le capteur de gaz externe est un capteur ultrasonic d'écoulement latéral et de masse molaire ou bien une combinaison du capteur de gaz et d'un capteur ultrasonic d'écoulement et de masse molaire.

**9.** Dispositif selon la revendication 8, où l'échantillon de gaz est prélevé du point central du chemin de mesure d'écoulement et de masse molaire, en évitant ainsi toute différence additionnelle de retard de temps provoqué par la vitesse d'écoulement et/ou la direction d'écoulement.

**10.** Dispositif selon la revendication 8, où l'échantillon de gaz est prélevé à travers le filtre ou un filet ou une maille qui couvre la chambre latérale du montage du transducteur ultrasonic du capteur d'écoulement ultrasonic de courant principal, en évitant ainsi toute différence additionnelle de retard de temps provoqué par la vitesse d'écoulement et/ou la direction d'écoulement et réduisant ou évitant additionnellement une contamination des capteurs de courant latéral.

Figure 1

Figure 2

Figure 3

Figure 4

**EP 1 764 036 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0646346 A